# EUROPEAN PATENT APPLICATION

(11) **EP 4 353 240 A1**
(43) Date of publication of application: **17.04.2024**
(21) Application number: 22382983.9
(22) Date of filing: 14.10.2022
(51) Int. Cl.: A61K 31/7076, A61K 9/00, A61K 45/06, A61P 31/04

(54) **CLADRIBINE FOR USE IN PREVENTING AND/OR TREATING AN INFECTION CAUSED BY GRAM-NEGATIVE BACTERIA**

(71) Applicant: Selabtec Sciences, SLU, 08225 Terrassa (ES)
(72) Inventor: PÉREZ OZCÁRIZ, Sergio, TERRASSA (ES); CASTELLS BOLIART, Josep, TERRASSA (ES)
(74) Representative: Ponti & Partners, S.L.P

(57) **Abstract**

The present invention relates to cladribine or a pharmaceutically acceptable salt, hydrate, solvate thereof for use in preventing and/or treating a bacterial infection, wherein said bacterial infection is caused by a gram negative bacteria.

## Description

### FIELD OF THE INVENTION

The present invention relates to the field of purine analogues. In particular the present invention relates to the purine analogue, cladribine, for use in preventing and/or treating a bacterial infection wherein said bacterial infection is caused by a gram negative bacteria.

### BACKGROUND

Infectious diseases are a major concern for global human health, according to WHO, due to the continuous resistance mechanisms that are being acquired by bacteria, specially by gram negative bacteria. Up to now, different groups of molecules have successfully been employed for the control of bacterial infections, such groups include different β-lactam antibiotics, macrolides, aminoglycosides, tetracyclines or flouroquinolones, among others. However, emerging drug resistance mechanisms are turning again bacterial infections in life-threatening diseases.

Nucleoside analogues are an extensive group of molecules that have been used as antiviral or anticancer drugs. Some nucleoside analogues have been studied to be used as antibacterial agents; however, very dissimilar results have been obtained. Inventors have surprisingly discovered that cladribine, although bacterial infections are described as side effects when administered for other indications, can be used for fight against some pathogenic bacteria, including some drug resistant bacteria, showing good potencies.

Cladribine (2-chloro-2'-deoxyadenosine [2-CdA]) is a purine analogue that selectively targets and suppresses lymphocytes implicated in the underlying pathogenesis of multiple sclerosis (MS) and B-cell leukaemia. Chemically, it mimics the nucleoside adenosine. However, unlike adenosine, it is relatively resistant to breakdown by the enzyme adenosine deaminase (ADA), which causes it to accumulate in targeted cells and interfere with the cell's ability to process DNA.

### SUMMARY OF THE INVENTION

In a first aspect, the present invention relates to cladribine or a pharmaceutically acceptable salt, hydrate, solvate thereof for use in preventing and/or treating a bacterial infection, wherein said bacterial infection is caused by a gram negative bacteria

### DETAILED DESCRIPTION

In a first aspect, the present invention relates to cladribine or a pharmaceutically acceptable salt, hydrate, solvate thereof for use in preventing and/or treating a bacterial infection, wherein said bacterial infection is caused by a gram negative bacteria. Alternatively, the present disclosure further relates to a method of preventing and/or treating a bacterial infection comprising the step of administering a therapeutically effective amount of cladribine or a pharmaceutically acceptable salt, hydrate, solvate thereof, wherein said bacterial infection is caused by a gram negative bacteria

In a further preferred embodiment, said gram negative bacteria belong to the genus *Serratia* or *Neisseria.*

As mentioned above, cladribine (2-chloro-2'-deoxyadenosine [2-CdA]) is a purine analogue that selectively targets and suppresses lymphocytes implicated in the underlying pathogenesis of multiple sclerosis (MS) and B-cell leukaemia. Chemically, it mimics the nucleoside adenosine.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

As used herein, the term "treat" and its cognates refer to an amelioration of a disease or disorder, or at least one discernible symptom thereof. In another embodiment, "treat" refers to an amelioration of at least one measurable physical parameter, not necessarily discernible by the patient, in another embodiment, "treat" refers to inhibiting the progression of a disease or disorder, either physically (e.g., stabilization of a discernible symptom), physiologically (e.g., stabilization of a physical parameter), or both, in another embodiment, "treat" refers to slowing the progression or reversing the progression of a disease or disorder. As used herein, "prevent" and its cognates refer to delaying the onset or reducing the risk of acquiring a given disease or disorder.

As used herein, the term "preventing and/or treating" includes "preventing and treating" and "preventing or treating".

The terms "therapeutically effective dose" and "therapeutically effective amount" are used to refer to an amount of a compound that results in prevention, delay of onset of symptoms, or amelioration of symptoms of a condition. A therapeutically effective amount may, for example, be sufficient to treat, prevent, reduce the severity, delay the onset, and/or reduce the risk of occurrence of one or more symptoms of a disorder associated with elevated ammonia concentrations. A therapeutically effective amount, as well as a therapeutically effective frequency of administration, can be determined by methods known in the art and discussed below.

It should be noted that the term "approximately" or "about" applied to the values used earlier and later in this document includes a margin of error of ± 5 %, such as, for example, ± 4 %, ± 3 %, ± 2 %, ± 1 %.

The present invention also relates to a pharmaceutical composition comprising cladribine for use in preventing and/or treating a bacterial infection according to the embodiments included herein. The composition may further comprise a pharmaceutically acceptable vehicle, adjuvant, diluent or excipient, such as preserving agents, fillers, disintegrating agents, wetting agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The present invention also relates to a method of preventing and/or treating a bacterial infection according to the embodiments included herein comprising the step of administering a therapeutically effective amount of cladribine or a pharmaceutically acceptable salt, hydrate, solvate thereof to a patient in need thereof. The term "patient" or "patient in need thereof", is intended for a human or non-human mammal affected or likely to be affected with a bacterial infection. Preferably, the patient is a human.

The compound of the present invention is capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition. The compound as provided herein can be formulated into pharmaceutical compositions by admixture with one or more pharmaceutically acceptable vehicle, adjuvant, diluent or excipient. Such compositions may be prepared for use in oral administration, particularly in the form of tablets or capsules; or parenteral administration, particularly in the form of liquid solutions, suspensions or emulsions.

In a preferred embodiment, the cladribine for use in preventing and/or treating a bacterial infection according to the embodiments included herein or a pharmaceutical composition comprising thereof is orally administered.

In another preferred embodiment, the cladribine for use in preventing and/or treating a bacterial infection according to the embodiments included herein or a pharmaceutical composition comprising thereof is parenterally administered, preferably intravenously administered.

In the case of oral administration, the tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bimodal.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

A number of examples will be provided below that are intended to illustrate the invention and in no way limit the scope of the invention, which is established by the attached claims.

### EXAMPLES

### Example 1: Growth inhibition over Pseudomonas aeruginosa

A fresh active culture of *Pseudomonas aeruginosa* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Low antimicrobial activity was detected at all concentrations.

### Example 2: Growth inhibition over Klebsiella pneumoniae

A fresh active culture of *Klebsiella pneumoniae* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity was detected at a cladribine concentration of 128 µg/mL.

### Example 3: Growth inhibition over Escherichia coli

A fresh active culture of *Escherichia coli* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity was detected at a cladribine concentration of 64 µg/mL, or higher.

### Example 4: Growth inhibition over Acinetobacter baumannii

A fresh active culture of *Acinetobacter baumannii* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Low antimicrobial activity was detected at all concentrations.

### Example 5: Growth inhibition over Enterobacter aerogenes

A fresh active culture of *Enterobacter aerogenes* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity was detected at a cladribine concentration of 128 µg/mL.

### Example 6: Growth inhibition over Salmonella typhimurium

A fresh active culture of *Salmonella typhimurium* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity was detected at a cladribine concentration of 64 µg/mL, or higher.

### Example 7: Growth inhibition over Shigella flexneri

A fresh active culture of *Shigella flexneri* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity was detected at a cladribine concentration of 128 µg/mL.

### Example 8: Growth inhibition over Proteus mirabilis

A fresh active culture of *Proteus mirabilis* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Low antimicrobial activity was detected at all concentrations.

### Example 9: Growth inhibition over Serratia marcescens

A fresh active culture of *Serratia marcescens* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity of cladribine was detected over *Serratia marcescens* at a concentration of 32 µg/mL.

### Example 10: Growth inhibition over Burkholderia cepacia

A fresh active culture of *Burkholderia cepacia* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Low antimicrobial activity was detected at all concentrations.

### Example 11: Growth inhibition over Neisseria meningitidis

A fresh active culture of *Neisseria meningitidis* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

Antimicrobial activity of cladribine was detected over *Neisseria meningitidis* at a concentration of 16 µg/mL.

### Example 12: Growth inhibition over Staphylococcus aureus

A fresh active culture of *Staphylococcus aureus* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

No antimicrobial activity was detected at any concentration.

### Example 13: Growth inhibition over Enterococcus faecium

A fresh active culture of *Enterococcus faecium* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

No antimicrobial activity was detected at any concentration.

### Example 14: Growth inhibition over Streptococcus pyogenes

A fresh active culture of *Streptococcus pyogenes* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

No antimicrobial activity was detected at any concentration.

### Example 15: Growth inhibition over Streptococcus pneumoniae

A fresh active culture of *Streptococcus pneumoniae* was inoculated in a 96 well microtiter plate in M-H liquid medium. In the same plate, serial dilutions of cladribine, starting at 128 µg/mL, were tested against the bacterial strain to determine the minimal cladribine concentration capable of inhibit the bacterial growth, defined as 80 % of Abs₆₀₀ diminution compared to a strain growth control.

No antimicrobial activity was detected at any concentration.

### Example 16: Growth inhibition over Candida albicans

A fresh active culture of *Candida albicans* was inoculated in a 96 well microtiter plate in M-H liquid medium. In the same plate, serial dilutions of cladribine, starting at 128 µg/mL, were tested against the fungal strain to determine the minimal cladribine concentration capable of inhibit the fungal growth, defined as 80 % of Abs₆₀₀ diminution compared to a strain growth control.

No antifungal activity was detected at any concentration.

### Example 17: Growth inhibition over Cryptococcus neoformans

A fresh active culture of *Cryptococcus neoformans* was inoculated in a 96 well microtiter plate in M-H liquid medium. In the same plate, serial dilutions of cladribine, starting at 128 µg/mL, were tested against the fungal strain to determine the minimal cladribine concentration capable of inhibit the fungal growth, defined as 80 % of Abs₆₀₀ diminution compared to a strain growth control.

No antifungal activity was detected at any concentration.

### Example 18: Growth inhibition over Aspergillus fumigatus

A fresh active culture of *Aspergillus fumigatus* was inoculated in a 96 well microtiter plate in M-H liquid medium. In the same plate, serial dilutions of cladribine, starting at 128 µg/mL, were tested against the fungal strain to determine the minimal cladribine concentration capable of inhibit the fungal growth, defined as 80 % of Abs₆₀₀ diminution compared to a strain growth control.

No antifungal activity was detected at any concentration.

### Example 19: Growth inhibition of several drug resistant strains of Neisseria meningitidis

MICs were determined by broth microdilution following the procedures recommended by the Clinical and Laboratory Standards Institute.

Serial two-fold dilutions of each compound were prepared in Microtiter plates (one for each strain). The concentration of the compounds SA-5 (Floxuridina) and SA-39 (Cladribine) was ranging from 128 to 0.25 µg/mL. The concentration of CIP was ranging from 16 to 0.03 µg/mL, respectively. Sterility and growth controls were included in all plates.

The bacterial suspensions were prepared and normalized to 0.5 McFarland (bioMérieux) turbidity standard (≈10⁸ CFU/ml) using physiological saline solution (sodium chloride 0.9%) (B. BRAUN). A 1/1000 dilution (≈10⁵ CFU/mL) of the suspensions were prepared in Todd-Hewitt Broth (Oxoid). The dilutions were inoculated to all columns except to the sterility control one. Only media was dispensed in the sterility control column. Inoculated plates were incubated 18-20h at 37°C.

ATCC reference strain was used as a control strain.

MIC values were considered as the concentration of compounds at which the growth was inhibited ≥80%, compared to growth control.

Results are shown in the following table 1:

**Table 1:**

| | **MIC** | |
|---|---|---|
| **STRAIN** | **SA-5** | **SA-39** |
| ***N*. *meningitidis ATCC*** | >128 | 16 |
| ***N. meningitidis 3*** | >128 | 16 |
| ***N. meningitidis 4*** | >128 | 32 |
| ***N*. *meningitidis 5*** | >128 | 16 |
| ***N. meningitidis 6*** | >128 | 16 |
| ***N*. *meningitidis 7*** | >128 | 16 |
| ***N. meningitidis 8*** | >128 | 16 |
| ***N. meningitidis 9*** | >128 | 16 |

As shown in Table 1, MIC values of cladribine (SA-39) are very stable independently of the strain, showing a potency in the range of 16 µg/mL. On the other side, the related compound floxuridine (SA-5) doesn't show any antimicrobial capacity even at the maximum assayed concentration of 128 µg/mL. (ATCC refers to ATCC 53417)

Table 2 shows the drug resistance profile of the assayed strains. Minimum inhibitory concentration (MIC) is expressed in µg/ml (left column). Clinical categorization (CC) (right column): R (resistant), I (susceptible, increased exposure) and S (susceptible). The clinical breakpoints according to EUCAST (European Committee on Antimicrobial Susceptibility Testing.) Ampicillin (AMP), amoxicillin-clavulanic acid (AMC), aztreonam (ATM), cefepime (FEP), cefotaxime (CTX), ceftriaxone (CRO), cefuroxime (CXM), ciprofloxacin (CIP), clarithromycin (CLR), chloramphenicol (CHL), erythromycin (ERY), levofloxacin (LVX), meropenem (MEM), moxifloxacin (MXF), penicillin (PEN), rifampin (RIF), tetracycline (TET) and and trimethoprim-sulfamethoxazole (SXT).

**Table 2:**

| | **STRAIN 3** | | **STRAIN 4** | | **STRAIN 5** | | **STRAIN 6** | | **STRAIN 7** | | **STRAIN 8** | | **STRAIN 9** | |
|---|---|---|---|---|---|---|---|---|---|---|---|---|---|---|
| | CMI | CC | CMI | CC | CMI | CC | CMI | CC | CMI | CC | CMI | CC | CMI | CC |
| **AMP** | - | - | - | - | **0.12** | **s** | - | - | - | - | - | - | - | - |
| **AMC** | - | - | - | - | <=0.05 | s | - | - | - | - | - | - | - | - |
| **ATM** | - | - | - | - | <=**0.012** | **s** | - | - | - | - | - | - | - | - |
| **FEP** | - | - | - | - | <=**0.25** | **s** | - | - | **0,004** | **s** | 0,038 | s | 0.006 | S |
| **CTX** | <**0.002** | **s** | - | - | 0,016 | s | - | - | - | - | - | - | - | - |
| **CRO** | - | - | - | - | <=0.25 | s | - | - | - | - | - | - | - | - |
| **CXM** | - | - | - | - | 0,5 | s | - | - | - | - | - | - | - | - |
| **CIP** | 0,03 | S | 0,03 | S | 0,03 | s | 0,03 | S | 0,03 | S | 0,03 | s | 0,03 | S |
| **CLR** | - | - | - | S | <=0.12 | s | - | S | - | S | - | s | - | - |
| **CHL** | - | - | - | - | <=2 | s | - | - | - | - | - | - | - | - |
| **ERY** | - | - | - | - | **<=0.06** | **s** | - | - | - | - | - | - | - | - |
| **LVX** | 0.004 | S | - | - | <=0.5 | s | - | - | - | - | - | - | - | - |
| **MEM** | **0.12** | **s** | - | - | <=**0.25** | **s** | - | - | - | - | - | - | 0,032 | - |
| **MXF** | - | - | - | - | <=0.25 | s | - | - | - | - | - | - | - | - |
| **PEN** | - | - | 0.125 | R | 0.06 | s | 0.19 | R | 0.125 | R | 0.38 | R | 0.19 | I |
| **RIF** | 0.008 | S | - | S | <=0.5 | s | - | S | - | S | - | s | - | - |
| **TET** | | | - | - | - | - | - | - | - | - | - | - | 0.5 | S |
| **SXT** | | | - | R | 0 | I | - | S | - | R | - | S | - | - |

As shown in Table 2, most of the strains used show some acquired resistance mechanism against conventional antibiotics. However potency of cladribine is not affected by these resistance mechanisms.

### Example 20: Growth inhibition of several multi drug resistant (MDR) strains of Neisseria gonorrhoeae

MICs were determined by broth microdilution following the procedures recommended by the Clinical and Laboratory Standards Institute.

Serial two-fold dilutions of each compound were prepared in Microtiter plates (one for each strain). The concentration of the compounds SA-5 (Floxuridina) and SA-39 (Cladribine) was ranging from 128 to 0.25 µg/ml. The concentration of CIP was ranging from 16 to 0.03µg/ml, respectively. Sterility and growth controls were included in all plates.

The bacterial suspensions were prepared and normalized to 0.5 McFarland (bioMérieux) turbidity standard (≈10⁸ CFU/ml) using physiological saline solution (sodium chloride 0.9%) (B. BRAUN). A 1/1000 dilution (≈10⁵ CFU/ml) of the suspensions were prepared in Gonococcal (GC) Medium supplemented with isovitalex, NaCl, K₂HPO₄, KH₂PO₄, starch from corn and proteose-peptone n°3. The dilutions were inoculated to all columns except to the sterility control one. Only media was dispensed in the sterility control column. Inoculated plates were incubated 18-20h at 37°C.

ATCC reference strain was used as a control strain.

MIC values were considered as the concentration of compounds at which the growth was inhibited ≥80%, compared to growth control.

Results are shown in next table 3:

**Table 3:**

| | **MIC** | |
|---|---|---|
| **STRAIN** | **SA-5** | **SA-39** |
| ***N*. *gonorrhoeae ATCC 43069*** | >128 | 32 |
| ***N*. *gonorrhoeae 1*** | >128 | 64 |
| ***N*. *gonorrhoeae 2*** | >128 | 32 |
| ***N. gonorrhoeae 3*** | >128 | 64 |
| ***N*. *gonorrhoeae 4*** | >128 | >128 |
| ***N*. *gonorrhoeae 5*** | >128 | 32 |
| ***N*. *gonorrhoeae 6*** | >128 | 64 |
| ***N. gonorrhoeae 7*** | >128 | 32 |
| ***N. gonorrhoeae 8*** | >128 | 128 |
| ***N. gonorrhoeae 12*** | >128 | 64 |
| ***N*. *gonorrhoeae 13*** | >128 | 64 |
| ***N*. *gonorrhoeae 14*** | >128 | 32 |
| ***N. gononhoeae 15*** | >128 | 64 |
| ***N*. *gonorrhoeae 18*** | >128 | 128 |

As shown in Table 3, cladribine is effective against several drug-resistant strains of *Neisseria gonorrhoeae.* Surprisingly, the related compound Floxuridine (SA-5) doesn't show any antimicrobial capacity even at the maximum assayed concentration of 128 µg/mL.

Table 4 shows the drug resistance profile of the assayed strains. Minimum inhibitory concentration (MIC) is expressed in µg/ml (left column). Clinical categorization (CC) (right column): R (resistant), I (susceptible, increased exposure) and S (susceptible). The clinical breakpoints according to EUCAST. Azithromycin (AZM), Ceftriaxone (CRO), Ciprofloxacin (CIP), Penicillin (PEN) and Tetracycline (TET).

| | **STRAIN 1** | | **STRAIN 2** | | **STRAIN 3** | | **STRAIN 4** | | **STRAIN 5** | | **STRAIN 6** | | **STRAIN 7** | | **STRAIN 8** | |
|---|---|---|---|---|---|---|---|---|---|---|---|---|---|---|---|---|
| | **MIC** | **CC** | **MIC** | **CC** | **MIC** | **CC** | **CMI** | **CC** | **MIC** | **CC** | **MIC** | **CC** | **MIC** | **CC** | **MIC** | **CC** |
| **AZN** | - | - | - | - | 0,064 | - | 1 | - | 0,38 | - | 0,032 | S | 0,25 | S | - | S |
| **CRO** | 0.006 | S | 0.006 | S | 0.004 | S | 0,002 | S | 0,008 | S | <0,016 | S | 0,094 | S | 0,047 | S |
| **CIP** | **0.064** | **R** | **0,75** | **R** | **0,18** | **R** | **4** | **R** | 0,003 | S | **8** | **R** | **12** | **R** | **1** | **R** |
| **PEN** | **-** | **-** | **-** | **-** | **-** | **-** | - | - | - | - | 0,25 | S | **1** | **I** | **0,25** | I |
| **TET** | **16** | **R** | **12** | **R** | **8** | **R** | 0,38 | S | 0,38 | S | 0,25 | S | **1,5** | **R** | **-** | S |

Table 5 shows the drug resistance profile of the assayed strains. Minimum inhibitory concentration (MIC) is expressed in µg/ml (left column). Clinical categorization (CC) (right column): R (resistant), I (susceptible, increased exposure) and S (susceptible). The clinical breakpoints according to EUCAST. Azithromycin (AZM), Ceftriaxone (CRO), Ciprofloxacin (CIP), Penicillin (PEN) and Tetracycline (TET).

| | **STRAIN 12** | | **STRAIN 13** | | **STRAIN 14** | | **STRAIN 15** | | **STRAIN 16** | | **STRAIN 17** | | **STRAIN 18** | |
|---|---|---|---|---|---|---|---|---|---|---|---|---|---|---|
| | **MIC** | **CC** | **MIC** | **CC** | **MIC** | **CC** | **MIC** | **CC** | **MIC** | **CC** | **MIC** | **CC** | **MIC** | **CC** |
| **AZM** | **2** | **R** | **4** | **R** | 0,25 | S | **1** | **R** | **1** | **R** | **2** | **R** | 0,19 | S |
| **CRO** | 0,008 | S | 0,004 | S | 0,004 | S | 0,005 | S | 0,012 | S | 0,016 | S | 0,032 | S |
| **CIP** | 0,006 | S | 0,04 | S | **2** | **R** | 0,012 | S | 0,016 | S | 0,006 | S | **0,5** | **R** |
| **PEN** | **0,125** | **I** | **0,19** | **I** | **>32** | **R** | **0,125** | **I** | **0,125** | **I** | **0,19** | **I** | **-** | **R** |
| **TET** | **1** | I | 0,5 | S | 0,25 | S | 0,5 | S | 0,5 | S | **1** | I | - | **R** |

As shown in Tables 4 and 5, most of the strains used show some acquired resistance mechanism against different groups of conventional antibiotics. However potency of cladribine is not affected by these resistance mechanisms and cross resistance is not detected.

## Claims

1. Cladribine or a pharmaceutically acceptable salt, hydrate, solvate thereof for use in preventing and/or treating a bacterial infection, wherein said bacterial infection is caused by a gram negative bacteria.

2. The cladribine for use according to claim 1, wherein said gram negative bacteria belong to the genus *Serratia* or *Neisseria.*

3. The cladribine for use according to any of claims 1 to 2, wherein said cladribine is administered by parenteral or oral route.

4. The cladribine for use according to any of claims 1 to 4, wherein said cladribine is included in a pharmaceutical composition.

5. The cladribine for use according to any of claims 1 to 4, wherein said pharmaceutical composition further comprises a supplementary active ingredient.
